(19) Europäisches Patentamt
European Patent Office
Office européen des brevets

(11) **EP 1 918 696 A2**

(12) **EUROPÄISCHE PATENTANMELDUNG**

(43) Veröffentlichungstag:
**07.05.2008 Patentblatt 2008/19**

(51) Int Cl.:
*G01N 22/04* (2006.01)     *G01N 33/38* (2006.01)

(21) Anmeldenummer: **07017835.5**

(22) Anmeldetag: **12.09.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **02.11.2006 CZ 20060692**

(71) Anmelder: **Vysoke uceni technicke v Brne Fakulta stavebni Ustav pozemniho stavitelstvi 602 00 BRNO (CZ)**

(72) Erfinder:
• **Skramlik, Jan**
  **6210 Brno (CZ)**
• **Moudry, Ivan**
  **61500 Brno (CZ)**
• **Stastnik, Stanislav**
  **63500 Brno (CZ)**
• **Novotny, Miloslav**
  **63500 Brno (CZ)**

(74) Vertreter: **Malusek, Jiri Kania, Sedlak, Smola Mendlovo namesti 1 a 602 00 BRNO (CZ)**

(54) **Verfahren zur Ermittlung der Feuchtigkeitsbewegung in porösen Stoffen und Vorrichtung zu derer Durchführung**

(57)    Die Vorrichtung für die Bestimmung des Feuchtigkeitstransports in porösen Stoffen wo nach dem Aufhängen der Probe zur Waage unter sie ein Behälter mit der Flüssigkeit mittels eines Verstellungsmechanismus angebracht wird, nachdem zur Probe die Mikrowellenstrahlung durch Sendewellenleiter zugeleitet wird und die Strahlung lässt man durch die Probe beim ihrer Stirn durchgehen und nach der Einschaltung des Verstellungsmechanismus werden der Sendewellenleiter und der Empfangswellenleiter kontinuierlich in Richtung oben längs der Probe geschoben und die Leiter messen kontinuirlich die Feuchtigkeit in dem Saturationsgebiet vom dem Stirn der Probe hinauf, wobei die Intensitätsänderung des Strahlungsdurchgangs und Gewichtsänderung der Probe in Zeit gemessen werden und die Ergebnisse quantifiziert und in einem angeschlossenem Personalcomputer weiter ausgewertet werden. Die Vorrichtung besteht aus einem Positioniermechanismus (3) für die Höhenverstellung des Behälters (2), und damit auch des Flüssigkeitsspiegels, wobei die Aufhängung (4) im oberen Bereich an einer Digitalwaage (5) befestigt ist, die über sie befestigt ist, wobei über dem Behälter (2) ein Sendewellenleiter (8) der Mikrowellenstrahlung angeordnet ist, an dessen Rande, der vom Behälter (2) abgewendet ist, eine Mikrowellenstrahlungsquelle (15) angeordnet ist, die an eine Spannungsquelle (6) angeschlossen ist, wobei auf der anderen Seite gegenüber dem Sendewellenleiter (8) ein Empfangswellenleiter (9) in derselben Höhe angeordnet ist, wobei die beiden Wellenleiter als Einheit an einem Tragrahmen über eine Verstellungseinrichtung (14) verstellbar angeordnet sind, und die Wellenleiter aus einem Metallhohlprofil ausgeführt sind, und die Probe (16) zwischen den gegenseitig zugewandten Enden der Wellenleiter (8, 9) angeordnet ist, wobei am Empfangswellenleiter (9) auf der vom Behälter (2) abgewandten Seite ein an einen Multimeter (11) angeschlossener Mikrowellenempfänger (10) angeordnet ist

Fig. 1

**Beschreibung**

Technisches Bereich

Die Erfindung betrifft das Verfahren zur Ermittlung der Feuchtigkeitsbewegung in porösen Stoffen, insbesondere auf dem Prinzip der Nutzung der elektromagnetischen Mikrowellenstrahlung und die Vorrichtung zu derer Durchführung.

Stand der Technik

Die meisten Wandbaukonstruktionen sind gemauerte Konstruktionen aus stückförmigen Baustoffen mit Mörtel als Bindemittel. Ein beträchtlicher Teil der Bauschäden ist von unerwünschter Feuchtigkeitseinwirkung verursacht und begleitet. Die derzeitigen Trends im Bauwesen zielen auf den Einsatz von neuen, leichten Baustoffen hin, wobei immer größere Anforderungen an eine mangelfreie Funktion von Baukonstruktionen, insbesondere an Wärmedämmungsfähigkeit der Außenwand gelegt werden. Die Ermittlung der charakteristischen Materialparameter für die Beurteilung des Feuchtigkeitszustandes von Baukonstruktionen ist nach wie vor ein unzureichend geklärtes Thema beim Studium der Materialeigenschaften von porösen Baustoffen. Die erforderliche Größe ist der Kapillartransportkoeffizient, der von der Messung der Sättigungs- und Entsättigungskurve (Verlauf der Feuchtigkeitsaufnahme und Austrocknung) gemäß der Bewegung der Lage des Feuchtigkeitsprofils im nichtstationärem Zustand (d.h. vor Erreichung der kompletten Durchfeuchtung) abgeleitet wird.

Für diesen Zweck wird üblicherweise die gravimetrische Methode angewendet, die das grundlegende Verfahren zur quantitativen Bestimmung des Feuchtigkeitsgehaltes in Stoffen darstellt. Sie besteht darin, daß die zu untersuchende Probe von einer bestimmten Länge an dem Stirn angefeuchtet wird. Das Wasser steigt während einer bestimmten Zeit in der Probe auf. Danach wird die Probe in mehrere Segmente - Proben aufgeteilt, die einen unterschiedlichen Durchfeuchtungsgrad aufweisen. In den einzelnen Segmenten wird die Feuchtigkeit gemessen, und aufgrund der Ergebnisse wird die Feuchtigkeitsverteilungskurve der Probe bestimmt. Diese Methode ist allgemein durch Ungenauigkeit bei Handhabung der Proben belastet, und für wiederholte Messungen an einer bestimmten Probe für die Geltendmachung der Integralmethode zur Bestimmung des Kapillartransportkoeffizienten ist sie ungeeignet.

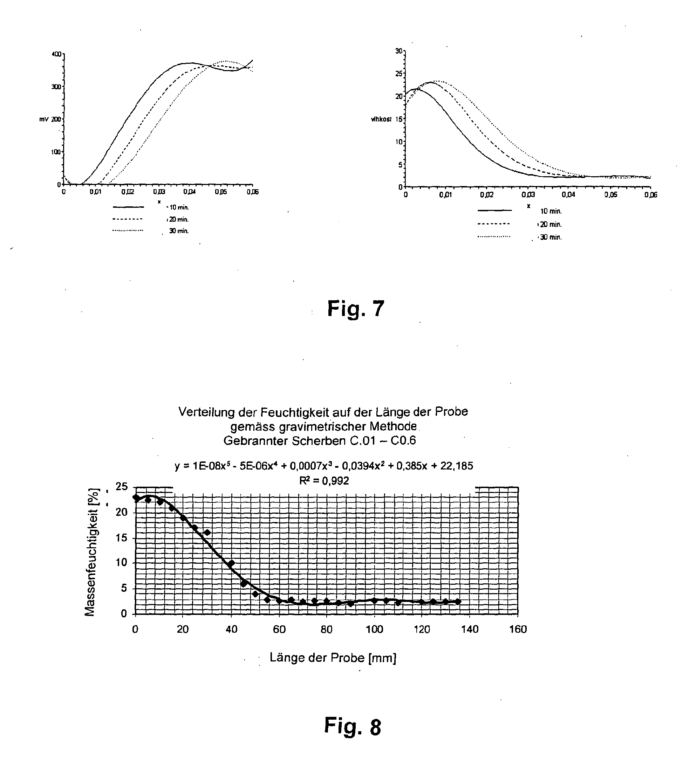

In Fig. 8 ist der Verlauf der Feuchtigkeitsaufnahmekurve bei der gravimetrischen Methode graphisch dargestellt. Es handelt sich um die Probe aus dem gebrannter Scherben.

Für die Feuchtigkeitsmessung könnte die Methode der Messung der Absorption von Neutronenstrahlung angewendet werden, die auf dem Prinzip der magnetischen Resonanz beruht. Dieses Verfahren ist allerdings sehr teuer und legt hohe Ansprüche an maschinelle Ausstattung sowie an Schutz vor gefährlicher Strahlung. Aufgrund dessen ist es für die Baupraxis nicht geeignet.

Ziel der Erfindung ist, ein Verfahren zur Ermittlung der Feuchtigkeitsbewegung in porösen Stoffen und eine Vorrichrung vorzustellen, die eine präzise und wiederholbare Messung ermöglichen würde, um die Integralmethode bei der Ermittlung des Kapillartransportkoeffizienten anwenden zu können, die relativ einfach und kostengünstig wäre.

Offenbarung der Erfindung

Die oben erwähnten Nachteile werden durch ein Verfahren zur Ermittlung der Feuchtigkeitsbewegung in porösen Stoffen gemäß der Erfindung behoben, deren Prinzip darin besteht, dass nach dem Aufhängen der Probe zur Waage unter sie ein Behälter mit der Flüssigkeit mittels eines Verstellungsmechanismus angebracht wird, nachdem zur Probe die Mikrowellenstrahlung durch Sendewellenleiter zugeleitet wird und die Strahlung lässt man durch die Probe beim ihrer Stirn durchgehen und nach der Einschaltung des Verstellungsmechanismus werden der Sendewellenleiter und der Empfangswellenleiter kontinuierlich in Richtung oben längst der Probe geschoben und die Leiter messen kontinuirlich die Feuchtigkeit in dem Saturationsgebiet vom dem Stirn der Probe hinauf, wobei die Intensitätsänderung des Strahlungsdurchgangs und Gewichtsänderung der Probe in Zeit gemessen werden und die Ergebnisse quantifiziert und in einem angeschlossenem Personalcomputer weiter ausgewertet werden.

Die Vorrichtung zur Ermittlung der Feuchtigkeitsbewegung in porösen Stoffen gemäß der Erfindung besteht aus einem Verstellungsmechanismus für die Höhenverstellung eines Behälters, und damit auch des Flüssigkeitsspiegels, wobei die Aufhängung in ihrem oberen Teil an einer Digitalwaage befestigt ist, die sich über die Aufhängung befindet, wobei über dem Behälter ein Sendewellenleiter der Mikrowellenstrahlung angeordnet ist, an dessen Rand, der von der zu untersuchten Probe abgewendet ist, eine Mikrowellenstrahlungsquelle angeordnet ist, die an eine Stromquelle angeschlossen ist, wobei ein Empfangswellenleiter in derselben Höhe auf der anderen Seite, gegenüber der Sendewellenleiter angeordnet ist, wobei die beiden Wellenleiter als Einheit an einem Tragrahmen über eine Verstellvorrichtung verstellbar angeordnet sind, und die Wellenleiter aus einem Metallhohlprofil ausgefertigt sind, und die Probe zwischen den beiden gegenseitig zugewendeten Enden der Wellenleiter angeordnet ist, wobei am Empfangswellenleiter ein an einen Multimeter angeschlossener Mikrowellenempfänger auf der von der zu untersuchenden Probe abgewandten Seite angeordnet ist.

In einer vorteilhaften Ausführung ist der Positioniermechanismus der Wellenleiter mit einem Elektromotor mit der Regelung der Geschwindigkeit der Wellenleiterverstellung entlang der Probe versehen.

Auf diese Art und Weise kann eine kontinuierliche Bewegung der Wellenleiter, und demzufolge auch eine kontinuierliche Messung gewährleistet werden.

In einer anderen vorteilhaften Ausführung stellt die Gunn-Diode die Mikrowellenstrahlungsquelle dar.

In einer weiteren vorteilhaften Ausführung sind in den beiden Wellenleitern Blenden für die Regelung der Strahlungsintensität eingebaut. Auf diese Art und Weise kann man die Strahlungsintensität entsprechend dem Charakter der Probe einfach ändern.

In einer weiteren vorteilhaften Ausführung sind alle Elemente an einem Rahmen angeordnet. Auf diese Art und Weise kann eine kompakte Einrichtung gebildet werden.

In einer anderen vorteilhaften Ausführung ist der Multimeter an einen Personalcomputer für eine digitale Visualisierung und Übertragung der Ergebnisse in Form von Kurven sowie zum Registrieren der Messwerte der Intensitätsänderung der elektromagnetischen Mikrowellenstrahlung in Abhängigkeit von der Massenfeuchtigkeit angeschlossen.

In einer weiteren vorteilhaften Ausführung ist die Digitalwaage ebenfalls an einen Personalcomputer angeschlossen, um die angezeigten Größen als kontinuierliche Erfassung des Gewichts der Probe während der Feuchtigkeitsaufnahme zu übertragen.

Kurze Beschreibung der Zeichnungen

Die Erfindung wird im Folgenden mit Hilfe von Zeichnungen vorgestellt. Es zeigen:

Fig. 1 - das Konstruktionsschema der Einrichtung gemäß der Erfindung,

Fig. 2 - der voraussichtliche Verlauf der Feuchtigkeitsaufnahme der Materialprobe

Fig. 3 - den grundlegenden Graphen mit den für die Ermittlung des Kapillartransportkoeffizienten benötigten Parametern,

Fig. 4 den Graphen, der aufgrund der Untersuchung einer konkreten Probe an der gegenständlichen Einrichtung gebildet wurde,

Fig. 5 den Graphen, der aufgrund der Messung des Gewichts der Probe während der Feuchtigkeitsaufnahme mit der gegenständlichen Einrichtung gebildet wurde,

Fig. 6 den Graphen der Ermittlung der Koordinate der Lage des Profils des Feuchtigkeitsfront in einem gewählten Zeitintervall vom Beginn der Feuchtigkeitsaufnahme,

Fig. 7 die Messoutputs, die im Maple-Programm verarbeitet wurden,

Fig. 8 den mittels gravimetrischer Methode, d.h. mit der Zerlegung, die im Stand der Technik beschrieben ist, ermittelten graphischen Verlauf der Feuchtigkeitsaufnahmekurve darstellt.

Ausführungsform der Erfindung

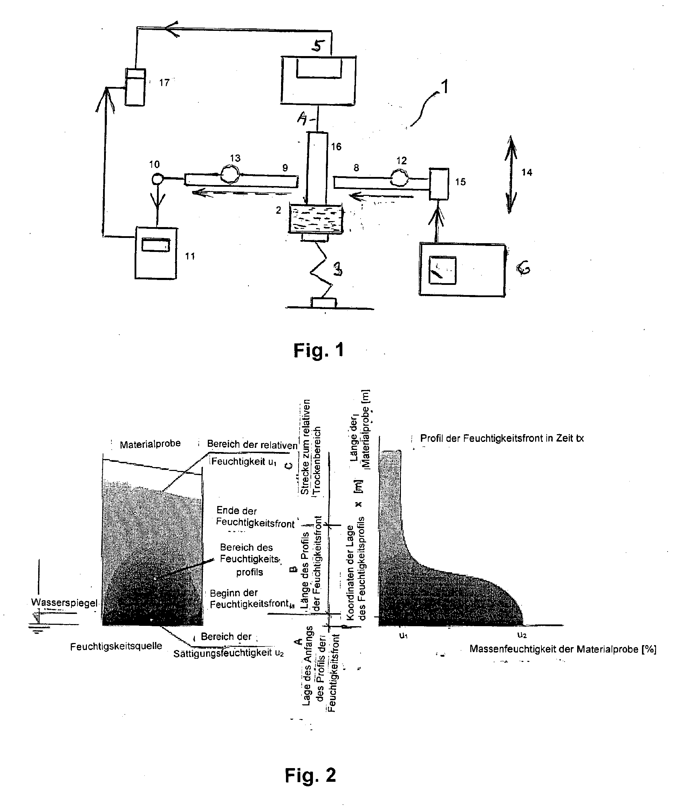

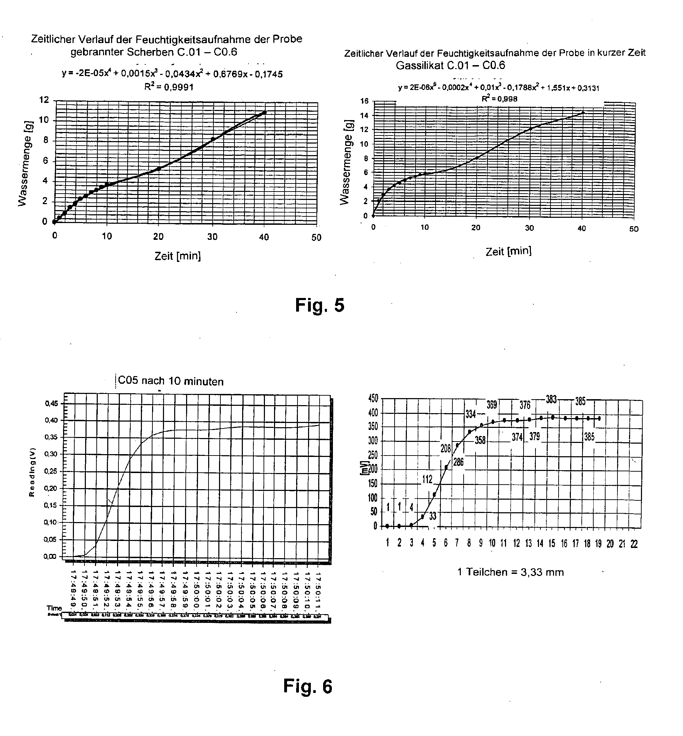

Aus dem Konstruktionsschema in der Fig. 1 ist ersichtlich, daß die Einrichtung 1 zur Ermittlung der Feuchtigkeitsbewegung in porösen Stoffen gemäß der Erfindung aus einem Behälter 2 für die Flüssigkeit, aus einem Positioniermechanismus 3, der zur Verstellung der Höhe des Behälters 2, und damit auch des Flüssigkeitsspiegels dient, besteht. Oberhalb des Behälters 2 befindet sich die zu untersuchende Probe 16, die auf einer Aufhängung aufgehängt ist, die an einer Digitalwaage 5 über die Probe aufgefangen ist. Im Bereich oberhalb des Behälters 2 ist ein Sendewellenleiter 8 der Mikrowellenstrahlung angeordnet, an dessen vom Behälter 2 abgewandten Rand sich eine Strahlungsquelle 15 befindet, in unserem Fall ist es die Gunn-Diode, die an eine Spannungsquelle 6 angeschlossen ist, die für die Funktion der Gunn-Diode erforderlich ist. Auf der anderen Seite vom Behälter ist gegenüber dem Sendewellenleiter 8 ein Empfangswellenleiter 9 angeordnet. Die gestrichelten Pfeile zeigen den Strahlungsfluss an. Die beiden Wellenleiter sind als Einheit mit einer Verstellvorrichtung 14 höhenverstellbar an einem nicht dargestellten Tragrahmen angeordnet. Die Verstellvorrichtung 14 ist mit einem Zweirichtungspfeil schematisch dargestellt. Die Wellenleiter 8 und 9 sind aus einem Metallhohlprofil ausgeführt. Auch die Waage 5 ist an dem nicht dargestellten Rahmen angeordnet. An den beiden Wellenleitern 8 und 9 sind Blenden 12 und 13 eingebaut, mit deren Hilfe die Strahlungsintensität geregelt werden kann. Die Probe 16 ist zwischen den gegenseitig zugewendeten Enden der Wellenleiter 8 und 9 angeordnet. Am Empfangswellenleiter 9 ist auf der vom Behälter 2 abgewendeten Seite ein Mikrowellenempfänger 10 angeordnet, der an den Multimeter 11 angeschlossen ist, in dem die Werte der Änderungen der Strahlungsintensität abgelesen werden können.

Der Multimeter ist an den Computer angeschlossen, in dem mit Hilfe eines entsprechenden Software-Programms die Ergebnisse, z.B. in Form von Kurven angezeigt werden können. Zur Übertragung der angezeigten Größen dient auch das Kommunikationsprogramm zur Datenablesung vom Bildschirm der Digitalwaage und das Software-Programm zu Multimeter 11.

Vor einer weiteren Beschreibung der Funktion der Einrichtung gemäß der Erfindung ist es sinnvoll, allgemeine Eigenschaften der untersuchten Werkstoffe in Erinnerung zu bringen. Bei der Befeuchtung des Stirnes der Probe 16 durch Kontakt mit dem Flüssigkeitsspiegel kommt es in einem bestimmten Zeitpunkt zur Aufsaugung der Feuchtigkeit in eine bestimmte Höhe der Probe. Dies ist sehr gut in der Fig. 2 dargestellt, in der der voraussichtliche Verlauf der Feuchtigkeitsaufnahme der Materialprobe dargestellt ist. Es handelt sich um ein sogenanntes Profil der Feuchtigkeitsfront. Die Feuchtigkeit in der Probe ist am höchsten am Flüssigkeitsspiegel und in Richtung nach oben entlang der Probe geht sie zurück. Diese sich ändernde Feuchtigkeit stellt den Untersuchungsgegenstand dar.

Zuerst ist der Feuchtigkeitsgefälle zu ermitteln. Falls die zeitliche und räumliche Verteilung der Feuchtigkeit, d.h. das sogenannte Feuchtigkeitsgefälle, bekannt ist, können aus den Messwerten die Werte des Kapillartransportkoeffizienten K ermittelt werden.

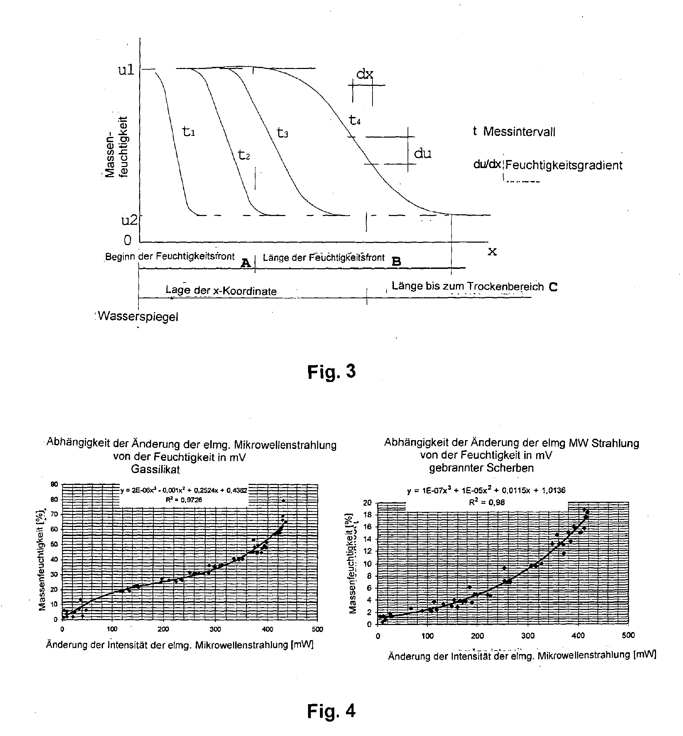

In Fig. 3 sind die Parameter dargestellt, die zur Ermittlung des Kapillartransportkoeffizienten erforderlich sind. A- Beginn der Feuchtigkeitsfront, B - Länge der Feuchtigkeitsfront, C - Länge bis zum Trockenbereich, D - Lage der x-Koordinate

Beim Einsatz einer nichtstationären Methode der Bestimmung des Kapillartransportkoeffizienten ist es sinnvoll, mehrere Kurven der Feuchtigkeitsaufnahme für verschiedene Zeitpunkte vom Anfang des Experimentes für eine und dieselbe Probe (Integralmethode) zu haben. Für die Matan-Methode reicht nur eine Feuchtigkeitsaufnahmekurve, die im nichtstationärem Zustand gebildet und aus den Messungen in ausgewählten Zeitintervallen sinnvoll ausgewählt wurde.

Für die Berechnung des Kapillartransportkoeffizienten ist die Methode von Matan üblich. Bei dieser Methode reicht es, eine Feuchtigkeitsaufnahmekurve zur Verfügung zu haben, die Zeit ab Beginn des Experimentes und die Koordinaten des Verlaufs der Feuchtigkeitsfront, die dieser Kurve entsprechen. Die Methode von Matan wendet die Boltzmanntransformation an, die bei kurzen Zeiten anwendbar ist, wo die Randbedingung am trockenen Ende der Probe noch nicht zur Geltung kommt.

Der Vereinfachung der Berechnung halber wurde die Funktionstransformation von zwei Variablen u, t zur Funktion einer Variablen $\eta$ angewendet:
Durch eine Boltzmanntransformation:

$$\eta = \frac{x}{2\sqrt{t}} \qquad\qquad (1)$$

$$(u(x,t) = \omega(\eta)\,,\ u(0,t) = u_1\,,\ u(x,0) = u_2) \qquad\qquad (2)$$

wird die Lösung der partialen Differentialgleichung zur Lösung einer gewöhnlichen Differentialgleichung in einer neuen Variablen $\eta$:

$$\frac{\partial}{\partial \eta} \cdot \left(\kappa(\omega) \cdot \frac{\partial \omega}{\partial \eta}\right) + 2\eta \frac{\partial \omega}{\partial \eta} = 0 \qquad\qquad (3)$$

mit Randbedingungen $w(0) = u_1\ \omega(\infty) = u_2$,
wo

$\Omega$    Neue Variable unter der Voraussetzung, daß t ein ausgewählter Zeitintervall ist

H     Transformation, bezeichnet als Boltzmannkoordinate [m.s-1/2]

$u_1$     Bestimmter zeitbedingter erreichter Wert der Massenfeuchtigkeit [-]

$u_2$     Massenfeuchtigkeit des Materials im stationären Zustand als relative Feuchtigkeit [-]

Bei bekannter Verteilung der Feuchtigkeit u(x) in der gegebener Zeit t, (d.h. t ist eine Konstante und u(x) ist die Funktion einer Variablen x) können wir die Gleichung (2) weiter umformen und den Kapillartransportkoeffizienten ermitteln:

$$\kappa(u(x)) = \frac{1}{2tu'(x)} \cdot \int_{x}^{\infty} \xi \cdot u'(\xi)d\xi \qquad\qquad (4)$$

k(u(x))     Kapittartransportkoeffizient, als Funktion der Feuchtigkeit von der Länge untersuchten Materialprobe in der Länge der Feuchtigkeitsfront [m$^2$.s$^{-1}$]

T     Zeitintervall, in dem die Feuchtigkeit als Funktion der Kurve u(x) in der Länge des Profils der Feuchtigkeitsfront gemessen wird [s]

Z     Substitution des entlang der Probe gemessenen Abstands vom Punkt an der Feuchtigkeitsaufnahmekurve du, die in der Gleichung bis ∞ ausgedrückt ist, für praktische Anwendung ist sie als Tor zum Intervall an der gemessnen Probe bis zum Abstand, wo die Feuchtigkeit den stationären Zustand erreichte, d.h. $u_2$, als Wert der relativen Feuchtigkeit des gemessenen Materials [-]

X     Koordinaten in der Länge der Probe und zur Identifizierung ihrer genauen Lage sind die Messwerte des Fortgangs der Feuchtigkeitsfront erforderlich, die ab der Ebene der unteren Fläche der gemessenen Probe feststellbar ist [m]

u'     Derivation der Feuchtigkeit gemäß der Raumkoordinate, anfangs wird sie °° sein und mit zunehmender Zeit wird sie zu 0 gehen. Durch die Substitution x durch ζ ist u' eine Derivation gemäß ζ [-]

Zur Ermittlung der Lage der Gewichtsfeuchtigkeit in der porösen Struktur einer Inertsubstanz wird die elektromagnetische Strahlung angewendet, die eine nicht destruktive und kontinuierliche Messung erlaubt, weist eine hohe Empfindlichkeit auf, und das Messergebnis wird von chemischer Zusammensetzung der Substanz bzw. der Menge des chemisch gebundenen Wassers nicht beeinflusst.
Die Mikrowellen dringen durch das Material unabhängig von seinen Eigenschaften durch. Zur Messung des Kapillartransportkoeffizienten in porösen Baustoffen ist eine Frequenz um $10^{10}$ Hz geeignet.
Bei Arbeit mit der Einrichtung gemäß der Erfindung wird wie folgt vorgegangen:

Bei Ermittlung der Feuchtigkeitsbewegung wird die Ermittlung der Intensitätsänderung der elektromagnetischen Mikrowellenstrahlung in Abhängigkeit von der Massenfeuchtigkeit im porösen Stoff angewendet. Es handelt sich um die Erfassung des eindimensionalen Transportprozesses in Laborbedingungen in Proben von Baustoffen bei ihrem direkten Kontakt mit dem Wasserspiegel. Die Erfassung der Änderungen der durch die Probe durchdringenden Intensität der elektromagnetischen Mikrowellenstrahlung dient zur Erfassung der Lage und Menge der im porösen Stoff enthaltenen Feuchtigkeit infolge einer synchronisierten zeitabhängigen Bewegung der Wellenleiter - der Sende- und Empfangsantenne entlang der untersuchten Baustoffprobe. Die in Zeit ausgedrückten ermittelten Werte des Positioniermechanismus der Wellenleiter können durch Umrechnung der Geschwindigkeit des Antriebs in Längenwerte übertragen werden, siehe Fig. 6.

Die Messapparatur ist an einen Personalcomputer angeschlossen. Zur Übertragung der angezeigten Größen dient ein Kommunikationsprogramm zum Datenablesen vom Bildschirm der Digitalwaage und die Software-Programm zum Multimeter.
Die Fig. 4 enthält einen Graphen, der bei Untersuchung einer konkreten Probe aus dem Gassilikat mit der gegenständlichen Einrichtung gebildet wurde und der die Abhängigkeit der Änderung der Intensität der elektromagnetischen Mikrowellenstrahlung von der Massenfeuchtigkeit in mV beim gebrannten Scherben darstellt. Es handelt sich um eine funktionelle Abhängigkeit der Änderung der Intensität der elektromagnetischen Mikrowellenstrahlung von der Massenfeuchtigkeit.

Die Fig. 5 enthält einen Graphen, der bei Untersuchung einer anderen konkreten Probe aus dem gebrannten Scherben mit der gegenständlichen Einrichtung gebildet wurde und stellt eine Kurve der Feuchtigkeitsaufnahme einer Gaskalkbetonprobe in Abhängigkeit von der Zeit dar.

In der Fig. 6 ist die Lage des Profils der Feuchtigkeitsfront im gewählten Zeitintervall vom Beginn der Feuchtigkeitsaufnahme sowie die Übertragung der Zeitangaben in Längendaten mit Hilfe einer synchronisierten Antriebseinheit und mit Benutzung des Linregrese-Exel-Programms graphisch dargestellt.

Die bei der Messung erfassten Werte können für die Ermittlung des Kapillartransportkoeffizienten mittels Verfahren herangezogen werden, die von der Bestimmung der Feuchtigkeitsverteilung (u/x,t) entlang der Probe in ausgewählten Zeitintervallen, d.h. von der Bestimmung der Feuchtigkeitsaufnahmekurven im nicht stationärem Zustand hervorgehen. Zur Feuchtigkeitsbestimmung an der gegebenen Stelle der Probe ist es sinnvoll, die Mikrowellenstrahlungsmessung heranzuziehen. Je höher die Feuchtigkeit des Materials ist, desto kleinere Menge an Mikrowellenstrahlung kann durch das Material durchdringen, denn die Wasserstoffkerne der Wassermoleküle absorbieren die Mikrowellenstrahlung. Aufgrund der Messung mittels Mikrowellenstrahlung können die Feuchtigkeit an der gegebenen Stelle des Materials ermittelt werden und die Feuchtigkeitsaufnahmekurven gebildet werden, die für die Ermittlung des Kapillartransportkoeffizienten K erforderlich sind.

Die einfachste Abhängigkeitsart ist die lineare Abhängigkeit. Die mit der zusammengestellten Apparatur gewonnenen Messwerte in Approximation durch ein Polynom k-ten Grades ermöglichen die Verarbeitung der Ergebnisse im Linregrese-Exel-Programm. Für weitere Berechnungen aus den Messwerten ist es sinnvoll, das mathematische Programmsystem Maple zu benutzen, das es ermöglicht, mit fast beliebig großen Ziffern zu arbeiten, Funktionsabhängigkeiten, Mengen usw. einfach zu definieren, Punktdaten, Kurven, Flächen unterschiedlich darzustellen, Systeme von Linear-, algebraischen und Differenzialgleichungen zu lösen und Berechnungen mithilfe von numerischen Methoden durchzuführen. In Fig. 7 sind die im Maple-Programm bearbeitete Outputs der Messungen dargestellt.

Industrielle Anwendung

Die Erfindung ermöglicht, Input-Daten für die Bestimmung des Kapillartransportkoeffizienten von porösen Baustoffen bei Nutzung der Eigenschaften der elektromagnetischen Mikrowellenstrahlung zu ermitteln. Das Verfahren ist durch die Anfangs- und Randbedingungen der Feuchtigkeitsaufnahme vor Erreichung des stationären Zustandes gegeben, der bei einer Reihe von Baustoffen dem Zustand der vollständigen Wassersättigung entspricht. Im Vergleich zu bekannten Laborverfahren weist sie eine vergleichbare Genauigkeit, niedrigere technische Ansprüche an Ausstattung mit Geräten und an Schutz vor Nebenwirkungen auf. Die zusammengestellte Messapparatur ermöglicht außer der Ermittlung der Bewegung des Feuchtigkeitsprofis auch die Bestimmung der Menge an Wasser, das in den porösen Stoff beim Kapillaraufstieg durchdringt, sowie die Ermittlung der Eichungsabhängigkeit für die Überführung der Messwerte des elektrischen Potentials zur Darstellung der Funktionsabhängigkeit der Änderung der Intensität der elektromagnetischen Mikrowellenstrahlung in energetische Einheiten in mV, gegebenenfalls als Dämpfung in Dezibeln mithilfe von eingelegten geeichten Blenden 12 und 13 in Abhängigkeit von der Massenfeuchtigkeit der untersuchten Probe.

Der konkrete Vorteil der Messapparatur kann nicht nur in der Möglichkeit der Aufstellung einer Methodik für die Bestimmung des Feuchtigkeitstransports in porösen Stoffen mithilfe der beschriebenen Messeinrichtung, sondern auch in ihrer weiteren Nutzung bei der für praktische Zwecke durchgeführten Forschung der Feuchtigkeitsproblematik bei Baukonstruktionen gesehen werden.

Weitere Beispiele des Einsatzes der zusammengestellten Messapparatur sind folgende:

- Bestimmung des Feuchtigkeitstransports in Baustoffen bei wiederholter Feuchtigkeitsaufnahme und Austrocknung,

- Bestimmung der Feuchtigkeitsbewegung in Baustoffen unter Einfluss und Einwirkung des mineralsalzhaltigen Wassers,

- Bestimmung des Feuchtigkeitstransports in Baustoffen bei Anwendung von Verfahren zur Unterdrückung der Feuchtigkeitsverbreitung in Baukonstruktionen durch Trocknungsmethoden,

- Fassung von direkten Schlussfolgerungen zur Effizienz der vorgeschlagenen elektroosmotischen Methode in der Projektphase für konkrete Beispiele,

- experimentelle Bestimmung des Kapillaraufstiegs,

- Feuchtigkeitsverbreitung durch die Schnittstelle zwischen unterschiedlichen Baustoffen.

**Patentansprüche**

1. Verfahren für die Bestimmung des Feuchtigkeitstransports in porösen Stoffen wo man der Stirn der Probe eintaucht, **dadurch gekennzeichnet, dass** nach dem Aufhängen der Probe zur Waage unter sie ein Behälter mit der Flüssigkeit mittels eines Verstellungsmechanismus angebracht wird, nachdem zur Probe die Mikrowellenstrahlung durch Sendewellenleiter zugeleitet wird und die Strahlung lässt man durch die Probe beim ihrer Stirn durchgehen und nach der Einschaltung des Verstellungsmechanismus werden der Sendewellenleiter und der Empfangswellenleiter kontinuierlich in Richtung oben längst der Probe geschoben und die Leiter messen kontinuirlich die Feuchtigkeit in dem Saturationsgebiet vom dem Stirn der Probe hinauf, wobei die Intensitätsänderung des Strahlungsdurchgangs und Gewichtsänderung der Probe in Zeit gemessen werden und die Ergebnisse quantifiziert und in einem angeschlossenem Personalcomputer weiter ausgewertet werden.

2. Die Vorrichtung für die Bestimmung des Feuchtigkeitstransports in porösen Stoffen bestehend aus einem Feuchtigkeitsbehälter 1 und einer Aufhängung für die Probe, **dadurch gekennzeichnet, dass** sie ferner aus einem Positioniermechanismus (3) für die Höhenverstellung des Behälters (2), und damit auch des Flüssigkeitsspiegels besteht, wobei die Aufhängung (4) im oberen Bereich an einer Digitalwaage (5) befestigt ist, die über sie befestigt ist, wobei über dem Behälter (2) ein Sendewellenleiter (8) der Mikrowellenstrahlung angeordnet ist, an dessen Rande, der vom Behälter (2) abgewendet ist, eine Mikrowellenstrahlungsquelle (15) angeordnet ist, die an eine Spannungsquelle (6) angeschlossen ist, wobei auf der anderen Seite gegenüber dem Sendewellenleiter (8) ein Empfangswellenleiter (9) in derselben Höhe angeordnet ist, wobei die beiden Wellenleiter als Einheit an einem Tragrahmen über eine Verstellungseinrichtung (14) verstellbar angeordnet sind, und die Wellenleiter aus einem Metallhohlprofil ausgeführt sind, und die Probe (16) zwischen den gegenseitig zugewandten Enden der Wellenleiter (8, 9) angeordnet ist, wobei am Empfangswellenleiter (9) auf der vom Behälter (2) abgewandten Seite ein an einen Multimeter (11) angeschlossener Mikrowellenempfänger (10) angeordnet ist.

3. Die Vorrichtung gemäß dem Anspruch 2, **dadurch gekennzeichnet, dass** der Verstellmechanismus (14) der Wellenleiter (8, 9) mit einem Elektromotor mit Regelung der Verstellungsgeschwindigkeit versehen ist.

4. Die Vorrichtung gemäß dem Anspruch 2, **dadurch gekennzeichnet, dass** die Mikrowellenstrahlungsquelle eine Gunn-Diode (15) ist.

5. Die Vorrichtung gemäß dem Anspruch 2, **dadurch gekennzeichnet, dass** in den beiden Wellenleitern (8, 9) Blenden (12, 13) für die Regelung der Strahlungsintensität eingebaut sind.

6. Die Vorrichtung gemäß dem Anspruch 2, **dadurch gekennzeichnet, dass** alle Elemente an einem Rahmen angeordnet sind.

7. Die Vorrichtung gemäß dem Anspruch 2, **dadurch gekennzeichnet, dass** der Multimeter (11) an einen Personalcomputer (17) für die Visualisierung der Ergebnisse, z.B. in Form von Kurven, angeschlossen ist.

8. Die Vorrichtung gemäß dem Anspruch 2, **dadurch gekennzeichnet, dass** die Waage (5) ebenfalls an einen Personalcomputer (17) zur Übertragung der angezeigten Größen angeschlossen ist.

9. Die Vorrichtung gemäß dem Anspruch 2, **dadurch gekennzeichnet, dass** der Positioniermechanismus (3) eine genaue Abgrenzung und Justierung des Kontaktes der untersuchten Probe (16) mit dem Spiegel der Flüssigkeit im Behälter (2) ermöglicht.

**Fig. 1**

**Fig. 2**

**Fig. 5**

**Fig. 6**

**Fig. 3**

**Fig. 4**

Fig. 7

Verteilung der Feuchtigkeit auf der Länge der Probe
gemäss gravimetrischer Methode
Gebrannter Scherben C.01 – C0.6

$y = 1E\text{-}08x^5 - 5E\text{-}06x^4 + 0{,}0007x^3 - 0{,}0394x^2 + 0{,}385x + 22{,}185$

$R^2 = 0{,}992$

Fig. 8